# EUROPEAN PATENT APPLICATION

(11) **EP 3 735 892 A1**
(43) Date of publication of application: **11.11.2020**
(21) Application number: 19735908.6
(22) Date of filing: 03.01.2019
(51) Int. Cl.: A61B 5/00, A61B 5/1455, A61B 5/0205, A61B 5/11, G06F 17/40

(54) **SYSTEM AND METHOD FOR DIGITAL MONITORING OF SLEEP APNEA**

(30) Priority: 05.01.2018 BR 102018000306
(71) Applicant: Almeida, Tácito, 05506-030 São Paulo (BR)
(72) Inventor: Almeida, Tácito, 05506-030 São Paulo (BR)
(74) Representative: Patentanwälte und Rechtsanwalt Weiß, Arat & Partner mbB
(86) International application number: PCT/BR2019/050001
(87) International publication number: WO 2019/134022

(57) **Abstract**

A system and method for digital monitoring of sleep apnea using non-invasive means for evaluating and monitoring obstructive sleep apnea by counting the number of oxygen desaturations, comprising a sensor (11) installed on the patient's finger, formed of an oximeter and an actigraph, a mobile telecommunications terminal (13) provided with a first application for interaction with said sensor and a remote server (16) provided with software for the final processing of the set of values measured by said sensor, wherein communication among the sensor, mobile terminal and remote server is provided by radiofrequency links. In addition, it is possible to monitor the patient's heart rate, body position, respiratory rate, and snoring, which is captured by the microphone (14) of the mobile terminal and preprocessed therein. The result of the processing performed in the remote server can be shown in the form of a graph and/or report.

## Description

### Technical field

The present invention refers to a non-invasive system for the digital monitoring of obstructive sleep apnea. More specifically, it is intended for continuous night monitoring in order to account for the number of oxygen desaturations, defined as the occasions when there is a drop of 3% or more in the SpO2 value, in addition to accounting for information of other quantities related to sleep apnea.

### Background

Sleep Apnea Syndrome (SAS) is an important sleep disorder that causes recurrent episodes of complete (apnea) or partial (hypopnea) blockage of the upper airways during sleep. One of the metrics that quantify the severity of SAS is the Apnea-Hypopnea Index (AHI), which is the number of apnea and hypopnea events per hour, on average, during the sleep period.

SAS affects a significant portion of the population, both adult men and adult women, being higher in different subsets of the population, such as the obese or the elderly. It is worth noting that many cases go undiagnosed because routine clinical tests and blood tests do not detect SAS and the gold standard test for the diagnosis of sleep apnea, polysomnography, is an expensive test, with low availability and high rejection by the patient. Nevertheless, accurate SAS screening is important due to adverse consequences such as daytime hypersomnia, neurocognitive dysfunction, cardiovascular disease, metabolic dysfunction, and respiratory failure.

A system and method for tracking SAS is described in US 9,545,227, entitled *Sleep Apnea Syndrome (SAS) Screening Using Wearable Devices,* which uses sensors to detect at least one physiological signal, specifically ECG and accelerometer. It combines this data with patient-related information and provides a risk assessment, displaying green (low risk) or red (high risk) light on a smartphone screen. The data captured in the patient is transmitted via Bluetooth or any other type of wireless link and processed in the capture device itself, in an external equipment, in a smartphone or in a cloud computing system. The treatment of the detected signals can include: analysis in the time domain, analysis in the frequency domain, statistical analysis, non-linear analysis and analysis of the position of the patient's body. The method is limited to showing on the screen of the external equipment whether the risk is high or low, although the results (such as the average occurrence of apnea episodes) are not available. However, its functionality is limited since it does not use oximetry or the measurement of other quantities.

US 2006/0173257, entitled *Sleep Evaluation Method, Sleep Evaluation System, Operation Program for Sleep Evaluation System, Pulse Oximeter, and Sleep Support System* describes a system comprising an oximeter formed by a sensor installed on the patient's finger and a main unity (*main body*) installed in the patient's body, provided with memory, microprocessor and battery, as well as a triaxial accelerometer for determining the position of the patient's body. The data stored in the memory is transmitted via an interface to a PC. In addition to a basic version, which uses data from SpO2, heart rate and body position, the document describes a more elaborate version, with additional insertion of data regarding breathing air flow, patient's snoring noise, chest movement, abdominal movement, and leg movements. The method described consists in correlating the combined information of SpO2 and position to assess the occurrence of apnea episodes, with several types of graphs, such as a histogram of apnea episodes as a function of the patient's decubitus angle.

Among the drawbacks of such system, it can be cited the limitation of the patient's movements due to the fact that the main unit, which includes a display, is buckled to the patient's body; in addition, the interconnection between the system modules is made by means of cables, which further limits the aforementioned mobility.

### Objectives of the invention

In view of the above, a first objective of the invention is to provide a system for monitoring sleep apnea using non-invasive sensors installed on the patient.

Another objective is to provide a system that does not require specific facilities (such as outpatient clinics or hospitals), which can be used in any environment, including at home.

Another objective is to make the results available to users and doctors in an easy and common way.

Another objective is the provision of a sleep apnea monitoring tool incorporated into routine checkups.

Another objective is the provision of a system that, due to its ease of use, allows the monitoring of sleep apnea treatment.

Another objective is to provide a low-cost solution enabling the examination of sleep apnea in populations including low-income populations.

### General description

The aforementioned objectives, as well as others, are achieved through the provision of a system consisting of a sensor installed on the patient's finger, consisting of an oximeter and an actigraph, and, optionally, a respiratory effort measuring belt or other sensors, such as heart rate, body position, respiratory rate, snoring audio sensors, etc., which communicate with a mobile telecommunications terminal, such as a smartphone or equivalent, through radiofrequency links.

According to another feature of the invention, the processing of the signals captured by said sensors is performed on a remote server, which guarantees greater processing capacity, consistency of the results and traceability of the exams.

According to a further feature of the invention, the set of the signals captured also comprises the patient's snoring, which is captured by the smartphone microphone (or equivalent equipment).

According to another feature of the invention, signal processing comprises the calculation of the ODI (Oxygen Desaturation Index). To do this, the sensor transmits the oxygen saturation value once per second. A proprietary algorithm, which runs on the server, identifies oxygen desaturations caused by apnea events, and the ODI is based on the relationship between the number of oxygen desaturations and the exam registration time, a value that is displayed on the smartphone screen (or equivalent equipment).

### Brief description of the drawings

Further features and advantages of the invention will become more evident by describing a specific embodiment, given by way of a non limiting example, and the drawings that refer to it, in which:
Figure 1 illustrates the proposed system, in its general aspect.
Figure 2 shows the results' screen, shown on the smartphone's display (or equivalent equipment).
Figure 3 is a detail of the image on the smartphone's screen (or equivalent equipment).
Figure 4 exemplifies a model of the report, which provides the result of the examination.

### Detailed description

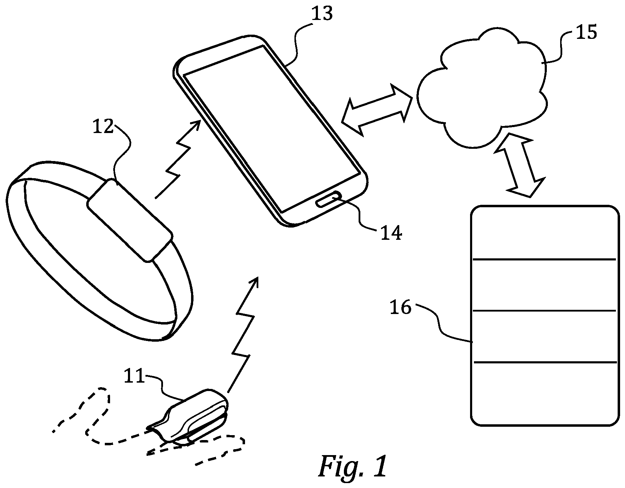

Referring now to Fig. 1, the system of the invention comprises a sensor 11 positioned on the patient's finger, which performs measurements of heart rate (HR), peripheral oxygen saturation (SpO2) and actimetry. The latter allows to determine whether the patient is awake or asleep, also helping to filter the SpO2 signal, since its measurement is very susceptible to the movement of the artifact.

The system also includes a respiratory effort belt 12, which captures data regarding chest distension due to the patient's breathing, as well as its position throughout the night's sleep. This belt includes an accelerometer that, by accelerating gravity on the x, y and z axes, detects the position of the patient's body.

The signals captured by said sensors are transmitted to the smartphone 13 via a radiofrequency link, preferably Bluetooth and more preferably Bluetooth Low Energy. Said smartphone is provided with a specific application that allows interaction with the sensors and, through the cloud 15, with a server 16 in which the software for the final processing of the set of measurements and generation of the exam results is installed.

According to the principles of the invention, the interface of the sensor 11 with the user is simplified, being limited to some LEDs that indicate the state of the sensor. The measured values are displayed on the smartphone screen from the moment its connection via Bluetooth is established.

During the exam, sensors 11 and 12 transmit the measured values to the smartphone at a rate of 1 Hz. In the event of a problem that prevents one or more of the aforementioned accesses, sensors 11 and 12 have memories with the capacity to store the data collected during the entire night of sleep, which can be transferred to the smartphone at the end of the exam period.

In addition to the data sent by said sensors, the smartphone can also analyze the patient's snoring audio, captured through its own microphone 14. In a preferred exemplary embodiment, the audio signal is divided into modular segments of one minute and each module is preprocessed through a specific program provided on the smartphone, using machine-learning resources. Said processing lasts less than 1 second and then a new module is processed and so on. The result of this processing is recorded in a file, much more compact than the original audio, to be transmitted to the server via the cloud 15.

At the end of the exam period, the measurements stored on the mobile terminal 13 are transmitted, via cloud 15, to the remote server 16 together with patient's identification data and responses to a pre-examination questionnaire presented on that user's smartphone. The software residing on the remote server comprises an algorithm for validating the data collected during the exam, also counting oxygen desaturations and other measured quantities in order to provide the final result comprising a report as well as the graphical representation of that result, displayed on the smartphone screen, said result being made available to the patient as well as to the doctor.

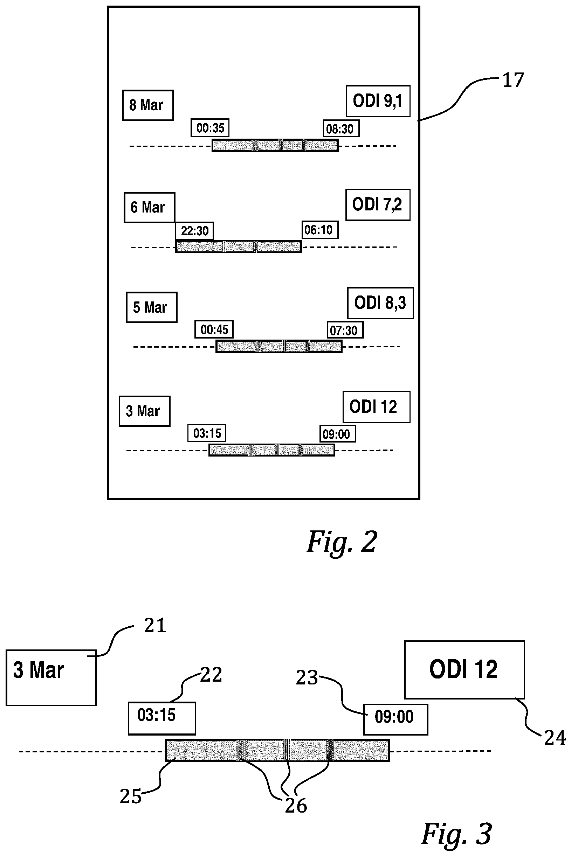

Fig. 2 illustrates the screen 17 of the smartphone, where the results of exams performed on different dates are displayed in the form of bars, with the events being color-coded. According to the detail of Fig. 3, the following information is provided for each of these exams:
- Exam date 21;
- Exam start time 22;
- Exam end time 23;
- Value of ODI 24;
- Representation of the exam duration using bar 25, and the occurrences of ODI 26 where a color scale is used to represent the amount of ODIs that occurred at each interval of, for example, 10 minutes. Thus, for example, the color red (not shown) indicates the occurrence of more than 20 ODIs in that range; the color yellow (not shown) indicates the occurrence of 10 to 20 ODIs and the color green (not shown) indicates the occurrence of 0 to 10 ODIs.

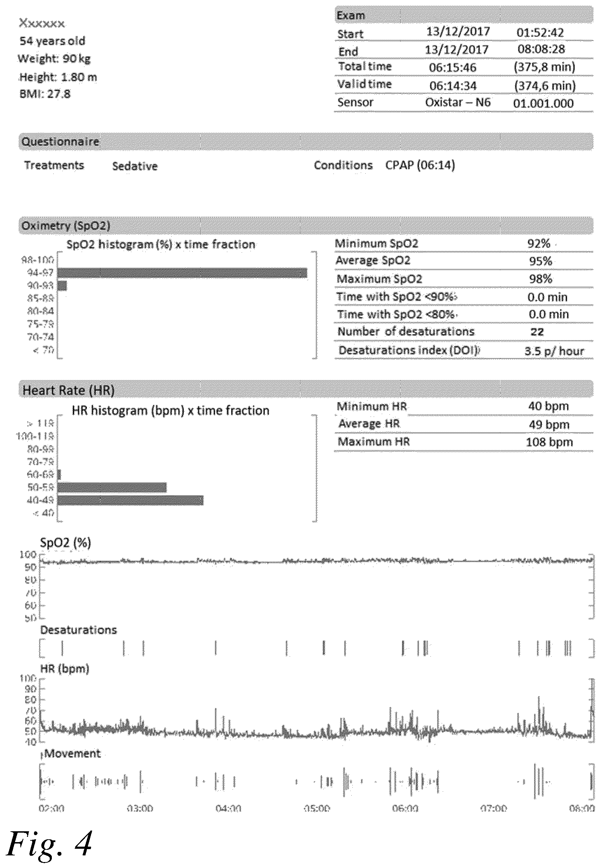

The exam result can also be made available in the form of a text report, as exemplified in Fig. 4. This report includes details of Oximetry and Heart Rate, as well as graphs that illustrate, according to time, oximetry (SpO2), heart rate (HR), the occurrence of desaturations and the patient's movement.

The server is also equipped with a high capacity memory, comprising a database where the results of the tests performed on different dates, corresponding to a plurality of patients, are stored. These tests can be sent to the patients' doctors, who will use them as an aid, both in the diagnosis and in the monitoring of the obstructive sleep apnea treatment.

## Claims

1. **SYSTEM FOR DIGITAL MONITORING OF SLEEP APNEA** based on non-invasive means for the assessment and monitoring of obstructive sleep apnea by counting the number of oxygen desaturations, in which there is a drop of 3% or more of the SpO2 value, comprising a sensor (11) installed on the patient's finger formed of an oximeter and an actigraph unit, a mobile telecommunications terminal (13) provided with a first application for interaction with said sensor and a remote server (16) provided with a software for the final processing of the set of values measured by said sensor, where the communication among the sensor, mobile terminal and remote server is provided by radiofrequency links.

2. **SYSTEM FOR DIGITAL MONITORING OF SLEEP APNEA,** according to claim 1, wherein the system additionally comprises one or more sensors from the group comprising a respiratory effort measuring belt, a heart rate sensor, a body position sensor, a respiratory rate sensor and a snoring audio sensor.

3. **SYSTEM FOR DIGITAL MONITORING OF SLEEP APNEA,** according to claim 2, wherein said snoring audio sensor comprises a microphone of a mobile telecommunications terminal (13).

4. **SYSTEM FOR DIGITAL MONITORING OF SLEEP APNEA,** according to claim 3, wherein said mobile telecommunications terminal comprises a specific program for preprocessing successive segments of said snoring audio, the result of which being transmitted to the server (16) via the cloud (15).

5. **SYSTEM FOR DIGITAL MONITORING OF SLEEP APNEA,** according to claim 4, wherein said segments last 1 minute.

6. **METHOD FOR DIGITAL MONITORING OF SLEEP APNEA** using the system as defined in claims 1 to 5, wherein the method comprises the transmission, to a mobile telecommunications terminal (13), through a radiofrequency link, of the signals captured by at least one sensor (11, 12, 14) positioned on or near a patient, followed by transmission, via the cloud (15), to a server (16) provided with a software for the final processing of said signals and the generation of the corresponding result.

7. **METHOD FOR DIGITAL MONITORING OF SLEEP APNEA,** according to claim 6, wherein said signals comprise the signals captured by a sensor (11) of peripheral oxygen saturation (SpO2), heart rate and actimetry.

8. **METHOD FOR DIGITAL MONITORING OF SLEEP APNEA,** according to claim 6, wherein said signals comprise the signals captured by a sensor (12) of respiratory effort and an accelerometer.

9. **METHOD FOR DIGITAL MONITORING OF SLEEP APNEA,** according to claim 6, wherein said signals comprise the signals captured by a microphone (14), said signals consisting of the patient's snoring audio.

10. **METHOD FOR DIGITAL MONITORING OF SLEEP APNEA,** according to claim 9, wherein the method comprises the preprocessing of successive segments of said patient's snoring audio signal through a program that uses machine learning resources, the results of said preprocessing being recorded in file and transmitted to a server (16).

11. **METHOD FOR DIGITAL MONITORING OF SLEEP APNEA,** according to any one of claims 6 to 10, wherein the method comprises the provision of the result of the processing of the data sent to the server (16) by the mobile telecommunications terminal (13) in a report and/or graphical means, or supplied to other systems through dedicated interfaces.

12. **METHOD FOR DIGITAL MONITORING OF SLEEP APNEA,** according to claim 11, wherein said report and/or graph comprise information regarding the exam date, exam start and end times, average ODI value and representation by means of bar indicating the occurrences of ODI at certain ranges.

13. **METHOD FOR DIGITAL MONITORING OF SLEEP APNEA,** according to claim 11, wherein said report comprises graphical representations, as function of time, SpO2, desaturation, heart rate and patient movement.
